# EUROPÄISCHE PATENTSCHRIFT

(11) **EP 1 406 919 B1**
(45) Veröffentlichungstag und Bekanntmachung des Hinweises auf die Patenterteilung: **15.08.2007**
(21) Anmeldenummer: 01957818.6
(22) Anmeldetag: 01.06.2001
(51) Int. Cl.: C07K 1/26

(54) **VERFAHREN ZUR ELEKTROPHORETISCHEN AUFTRENNUNG VON MEMBRANPROTEINEN**
METHOD FOR ELECTROPHORETICALLY SEPARATING MEMBRANE PROTEINS
PROCEDE DE SEPARATION ELECTROPHORETIQUE DE PROTEINES MEMBRANAIRES

(30) Priorität: 03.06.2000 DE 10027705
(43) Veröffentlichungstag der Anmeldung: 14.04.2004
(73) Patentinhaber: Nimbus Biotechnologie GmbH, 04317 Leipzig (DE)
(72) Erfinder: BAYERL, Thomas, 82110 Germering (DE); SACKMANN, Erich, 81827 München (DE)
(74) Vertreter: Patentanwälte Ruff, Wilhelm, Beier, Dauster & Partner
(86) Internationale Anmeldenummer: PCT/EP2001/006234
(87) Internationale Veröffentlichungsnummer: WO 2001/094421

(56) Entgegenhaltungen:
- XU CHONG ET AL: "Two-dimensional electrophoretic profile of human sperm membrane proteins." JOURNAL OF ANDROLOGY, Bd. 15, Nr. 6, 1994, Seiten 595-602, XP001034082 ISSN: 0196-3635
- STELZLE M ET AL: "Two-dimensional microelectrophoresis in supported lipid bilayers." BIOPHYSICAL JOURNAL, Bd. 63, Nr. 5, 1992, Seiten 1346-1354, XP001034091 ISSN: 0006-3495 in der Anmeldung erwähnt

## Beschreibung

Die zweidimensionale Auftrennung von Proteinen ist eine Voraussetzung für die Proteomanalyse von Zellen und erfolgt bisher fast ausschließlich durch die zweidimensionale Polyacrylamid-Gelektrophorese (2D-PAGE). Der Nachteil dieser Technik liegt in der Unlösbarkeit von Membranproteinen in den für die 2D-Page Technik verwendeten Gelen. Unter Membranproteinen sollen hier solche Proteine verstanden werden, die entweder die Membran durchspannen (z.B. lonophore oder Rezeptoren) oder zumindest einseitig in den hydrophoben Bereich der Membran eingebettet sind (z.B. Oberflächenrezeptoren). Damit ist die 2D-PAGE Technik nicht in der Lage, Membranproteine aufzutrennen. Da Membranproteine jedoch für die Funktion einer Zelle eine überragendende Bedeutung besitzen, ist die auf 2D-PAGE basierende Proteomanalyse unvollständig.

Zur Überwindung dieses Problems kommt es also darauf an, eine elektrophoresefähige Matrix zu finden, die eine Lösung des Membranproteins erlaubt und darüber hinaus auch noch das Anlegen von Ionengradienten oder pH-Gradienten zur weiteren Auftrennung gestattet.

Weitere Ansätze wurden in der Literatur beschrieben, die auf eine Lösung spezifischer 2D-PAGE Probleme abzielen (Wall DB, Kachman MT, Gong SY, et al. "Isoelectric focusing nonporous RP HPLC: A two-dimensional liquid-phase separation method for mapping of cellular proteins with identification using MALDI-TOF mass spectrometry" Anal. Chem. 72: (6) 1099-1111, 2000; Seehof K, Kresse M, Mader K, et al. "Interactions of nanoparticles with body proteins - improvement of 2D-PAGE-analysis by internal standard" Int. J. Pharm. 196: (2) 231-234, 2000) konnten jedoch für das erwähnte Löslichkeitsproblem integraler Membranproteine noch keine befriedigende Alternative aufzeigen.

Bereits Anfang der neunziger Jahre wurden Lipiddoppelschichten (im folgenden Bilayer genannt), die auf einem planaren Substrat aufgebracht waren (sogenannte festkörperunterstützte Bilayer), erfolgreich auf ihre Elektrophoresefähigkeit untersucht. Es konnte gezeigt werden, daß die Lipide des Bilayers entsprechend der elektrischen Ladung ihrer Kopfgruppe durch ein angelegtes elektrisches Feld innnerhalb der Ebene des Bilayers (d.h. zweidimensional) verschiebbar waren (Stelzle, M., Sackmann, E., "Sensitive detection of protein adsorption to supported lipid bilayers by frequencydependent capaciance measurements and microelectrophoresis", Biochim. Biophys. Acta 981, 135-142, 1989; Stelzle, M., Miehlich, R., Sackmann, E. "Two-dimensional microelectrophoresis in supported lipid bilayers", Biophysical Journal 63, 1346-1354, 1992; Groves J.T., Boxer S.G. "Electric field-induced concentration gradients in planar supported bilayers", Biophys J. 69, 1972-1975, 1995). Weitere Arbeiten zeigten, das auch die Mikrostrukturierung des Substrates (meistens Silizium oder Silikate) einen Einfluß auf die Bewegung und die Bewegungsrichtung der Lipide im elektrischen Feld hat (van Oudenaarden A., Boxer S.G., "Brownian ratchets: molecular separations in lipid bilayers supported on patterned arrays",Science. 285,1046-1048, 1999) und dieser Bewegung auch für die Lipide unüberwindliche Barrieren setzen kann (Groves J.T., Ulman N., Boxer S. G. "Micropatterning fluid lipid bilayers on solid supports", Science 275, 651-653, 1997).

Auch die Verwendung von Lipid-Monoschichten (im folgenden Monolayer genannt), die durch sogenannnten Langmuir-Blodgett Transfer auf planare Substrate übertragen wurden, als elektrophoresefähige Matrix ist in der Literatur beschrieben und Experimente konnten auch hier die Bewegung der Lipide in der Ebene des Monolayers durch die Wirkung des angelegten elektrischen Feldes demonstrieren (Dietrich, C., Tampé, R. "Charge determination of membrane molecules in polymer-supported lipid bilayers", Biochim. Biophys. Acta 1238, 183-191, 1995).

Eine Verwendung der lipidbasierenden Elektrophoresematrizen für die Auftrennung von Membranproteinen ist in der Literatur bisher nicht beschrieben. Prinzipiell sollten diese dafür geeignet sein, da die Lipide in Form der Lipiddoppelschicht (Bilayer) die natürliche Matrix für Membranproteine darstellen, und die Anwesenheit spezifischer Lipide häufig für die Funktion der in sie eingebetteten Membranproteine entscheidend ist.

Xu Chong et al. beschreiben die Charakterisierung von humanen Spermatozoenmembran-Proteinen mit Hilfe der zweidimensionalen Elektrophorese und Computer-Bild-Analyse (Xu Chong et al.: "Two-dimensional electrophoretic profile of human sperm membrane proteins." Journal of Andrology, Bd. 15, Nr. 6, 1994, Seiten 595 - 602). Die hochangereicherten Spermienmembranvesikel haben eine Doppellaminatstruktur, die für biologische Membranen typisch ist. Lipidbasierende Elektrophoresematrizen kommen dabei allerdings nicht zur Anwendung. Die Ausbildung eines planaren Bilayers aus den genannten Vesikeln auf einem Substrat wird nicht beschrieben.

Für Lipidmonolayer ist sofort offensichtlich, daß sie für eine solche Anwendung ungeeignet sind, da sie nur-eine halbe Membran darstellen und damit einem Membranprotein keine funktionelle Umgebung liefern können.

Für festkörperunterstützte Bilayer sind es im wesentlichen zwei Probleme, die eine Anwendung dieser Systeme als Elektrophoresematrix für Membranproteine bisher unmöglich machten.
1) Die bisher beschriebenen Präparationsmethoden für diese Bilayer sind kaum geeignet, um die gesamte Mannigfaltigkeit von Membranproteinen einer Zelle in einen festkörperunterstützten Bilayer zu inkorporieren. Dies ist bisher lediglich für einzelne Membranproteine über die Fusion von sogenannten Proteoliposomen auf der Substratoberfläche gelungen (Salafsky J., Groves J.T., Boxer S.G. "Architecture and function of membrane proteins in planar supported bilayers: a study with photosynthetic reaction centers", Biochemistry 26, 14773-14781, 1996).
2) Durch den engen Kontakt des Bilayers mit der anorganischen Substratoberfläche (die Dicke der Wasserschicht dazwischen beträgt maximal 3 nm) (Johnson S.J., Bayerl T.M., McDermott D.C., Adam G.W., Rennie A.R., Thomas R.K., Sackmann E. "Structure of an adsorbed dimyristoylphosphatidylcholine bilayer measured with specular reflection of neutrons", Biophys J. 59, 289-294, 1991; Krueger S., Koenig B.W., Orts W.J., Berk N.F., Majkrzak C.F., Gawrisch K. "Neutron reflectivity studies of single lipid bilayers supported on planar substrates", Basic Life Sci. 64, 205-213, 1996) kommt das Membranprotein der Substratoberfläche zu nahe und kann daran denaturieren oder immobilisiert werden. Damit ist seine Bewegung in der Ebene des Bilayers durch äußere elektrische Felder extrem behindert oder unmöglich.

Das neue Verfahren soll diese Probleme beheben und damit erstmals die Elektrophorese des gesamten Besatzes von Membranproteinen einer Zelle, Zellorganelle bzw. eines Gewebes aus gleichartigen Zellen mit einem Bilayer als Matrix ermöglichen.

Die erfindungsmäßige Umsetzung des neuen Verfahrens erfolgt in drei Schritten.
a) Durchführung einer geeigneten chemischen Modifizierung der Substratoberfläche mit dem Ziel, einen direkten Kontakt zwischen dem im festkörperunterstützten Bilayer befindlichen Membranprotein und Substrat zu verhindern und gleichzeitig eine Beweglichkeit des Membranproteins in der Ebene des auf diese modifizierte Oberfläche aufgebrachten Bilayers durch äußere elektrische Felder, pH-Gradienten oder Salzgradienten zu ermöglichen.
b) Aufbringung des Bilayers auf die modifizierte Oberfläche in einer Weise, daß weitgehend alle Membranproteine einer Zelle oder Zellorganelle übertragen werden und damit für die zweidimensionale Auftrennung zur Verfügung stehen.
c) Räumliche Auftrennung der Membranproteine durch Applikation geeigneter elektrischer Felder, pH-Gradienten oder Salzgradienten nach Methoden, die Stand der Technik sind.

Die Lösung des ersten Schrittes wird durch die chemische Modifizierung der Oberfläche mittels Verfahren erreicht, die Stand der Technik sind. Allgemein kann dieses Ziel auf zwei Wegen erreicht werden: (i) Durch die nichtspezifische Adsorption von Monomeren oder Polymeren nach Methoden, die Stand der Technik sind (insbesondere Deposition aus der Lösung). (ii) Durch kovalente Fixierung nach Stand der Technik der unter (i) genannten Moleküle auf der Substratoberfläche. Kriterium für die Auswahl der für (i) und (ii) verwendeten Moleküle ist die Ausbildung einer hydrophilen Grenzfläche zum Bilayer, die spontane Bildung eines Bilayers nach Methoden laut Stand der Technik (insbesondere Vesikelfusion) auf dieser modifizierten Oberfläche und die diffusive Bewegung der Bilayerkomponenten (insbesondere der darin befindlichen Membranproteine) in der Ebene des gebildeten Bilayers.

Die erfindungsmäßige Umsetzung des zweiten Schrittes erfolgt durch die Verwendung von Vesikeln aus natürlichem oder transgenem Zell- bzw. Gewebematerial für die Beschichtung der im ersten Schritt erzeugten Oberfläche mit einem Bilayer. Hierzu werden die Zellen zunächst durch Separationsmethoden, die Stand der Technik sind, aufgeschlossen und in ihre einzelnen Organellen getrennt. Aus der Suspension der interessierenden Organellen werden sodann nach Verfahren, die ebenfalls Stand der Technik sind, Vesikel hergestellt, die zu einer spontanen Bildung eines planaren Bilayers auf der modifizierten Oberfläche fähig sind. Die für die Präparation der Vesikel verwendeten Methoden (insbesondere die Behandlung des Membranmaterials mit Ultraschall und die Extrusion (Hochdruckfiltration) durch nanoporöse Filter) müssen hierbei sicherstellen, daß die in den Membranen befindlichen Membranproteine auch in den durch die Behandlung entstehenden Vesikeln enthalten sind.

Das Ergebnis der erfolgreichen Umsetzung beider Schritte ist eine planare Membran mit einer Zusammensetzung aus Lipiden und Proteinen (einschließlich und insbesondere von Membranproteinen), die weitgehend mit jener des verwendeten Zellmaterials übereinstimmt und deren Komponenten in der Ebene des Bilayers eine diffusive Beweglichkeit zeigen.

Im dritten Schritt wird dann durch Anlegen eines elektrischen Feldes in der Ebene des Bilayers, bei Bedarf unterstützt durch zusätzliche elektrische Steuerfelder, deren Orientierung (elektrischer Feldvektor) auch außerhalb dieser Ebene liegen kann, eine ein- oder zweidimensionale Auftrennung der Membranproteine entsprechend ihrer Ladung und Molekülgröße erreicht. Im Unterschied zur im Stand der Technik erwähnten 2D-PAGE Methode sind die Proteine jedoch schon vor Anlegen des elektrischen Feldes infolge der beschriebenen Präparation des Bilayers über die gesamte planare Oberfläche verteilt. Die anzulegenden elektrischen Felder müssen dieser Tatsache Rechnung tragen. Eine weitere Auftrennung der Membranproteine ist durch das Anlegen von pH-Gradienten oder Salzgradienten auf der zum Volumen orientierten Seite des Bilayers entsprechend Methoden, die Stand der Technik sind, möglich. Eine noch bessere Auftrennung ist durch die Applikation separater pH- oder Salzgradienten innerhalb der im ersten Schritt beschriebenen chemisch modifizierten Oberfläche, parallel zur Bilayerebene, möglich.

Schießlich kann auch noch eine zusätzliche Mikro- oder Nanostrukturierung des verwendeten Substrates die Auftrennung der Membranproteine mit den oben beschriebenen Methoden weiter verbessern. Diese Möglichkeit ist für Lipide in festkörperunterstützten Bilayern bereits Stand der Technik.

Eine weitere Analyse der ein- bzw. zweidimensional aufgetrennten Proteine ist möglich, indem nach der Elektrophorese der Träger mit der Membran aus der wäßrigen Phase, in die er bis dahin engebettet war, entfernt wird. Dies kann zum Beispiel durch eine rasche Trocknung von Träger mit Membran erfolgen, welche eine weitere Diffusion der Membrankomponenten (Lipide und Proteine) fast vollständig unterbindet. Damit wird die durch die Elektrophorese erzielte Auftrennung fixiert, eine Rückkehr in den Ausgangszustand ist nicht mehr möglich. Die weitere Analyse kann nun durch geeignete Verfahren hoher Detektionsempfindlichkeit, die Stand der Technik sind (insbesondere Fluoreszenz- und Lumineszenzdetektion sowie radioaktive Detektion von zuvor entsprechend markierten Membranproteinen) erfolgen.

### In den Abbildungen ist gezeigt:

Abb. 1: Schematische Darstellung (Seitenansicht) der zur Elektrophorese verwendeten festkörperunterstützten Membran auf einem modifizierten Träger. Die gesamte Membran befindet sich in Pufferlösung (nicht gezeichnet).
Abb. 2: Schematische Darstellung (Aufsicht) des verwendeten Meßaufbaus zur Durchführung der Elektrophorese an Membranproteinen. Die in Abb. 1 gezeigte Membran befindet sich in der Mitte zwischen den 4 Elektroden. Nicht gezeichnet ist die von oben aufsetzbare Kammer, welche den Aufbau wasserdicht versiegelt und über die Stutzen zum Befüllen mit Pufferlösung verfügt.
Abb. 3: Abhängigkeit der Position des Fluoreszenzminimums von Gykophorin (Quadrate) und Bande-3 Protein (Kreise) zwischen den Elektroden (x-Richtung) von der Dauer der Einschaltzeit tₑ eines elektrischen Feldes einer Feldstärke von 200 V/cm nach Beispiel 3.1.
Abb. 4: Abhängigkeit der Position des Fluoreszenzminimums der Kalzium ATP-ase des Sarkoplasmatischen Retikulums zwischen den Elektroden (x - Richtung) von der Dauer der Einschaltzeit tₑ eines elektrischen Feldes einer Feldstärke von 200 V/cm nach Beispiel 3.2.

### Beispiele

### 1. Modifikation von Festkörperoberflächen

Eine Aminomodifizierung eines Objektträgers mit Aminopropyltrimethoxysilan (EDA) und anschließende Beschichtung mit Polystyrolsulfonat (PSS) wurde folgendermaßen durchgeführt. In eine frisch hergestellte Silanlösung bestehend aus 0,5 ml EDA und 14 µl konzentrierter Essigsäure in 50 ml entionisiertem *Wasser wurde* für die Dauer von 2 Stunden ein Glasobjektträger für Mikroskopiezwecke der Größe 20 x 20 mm vollständig eingetaucht. Danach wurde das modifizierte Substrat dreimal mit entionisiertem Wasser gespült und eine Stunde bei 80°C getempert. Nach der Silanisierung des Substrats ändert sich das Benetzungsverhalten. Der Erfolg der Behandlung wurde mittels Kontaktwinkelmessungen nach Methoden, die Stand der Technik sind, dokumentiert.

Anschließend wurde das Glassubstrat für 30 Minuten in eine Na-Polystyrolsulfonat (PSS) - Lösung, bestehend aus 12,5 mg PSS in 25 ml entionisiertem Wasser, eingetaucht. Danach wurde das modifizierte Substrat dreimal mit entionisiertem Wasser gespült und getrocknet. Der Erfolg der Polymeradsorption wurde mittels FTIR-Spektroskopie im Reflexionsmodus dokumentiert.

### 2 Aufbringung einer natürlichen Membran auf die modifizierte Oberfläche

### 2.1 Erythrozytenmembran

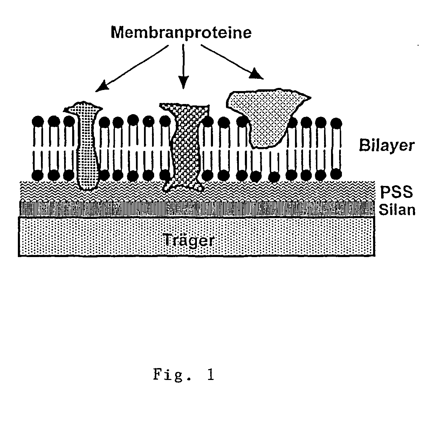

Aus menschlichen Erythrozythen wurden sogenannte "right-side-out" Vesikel (Ery-RSO-Vesikel) nach Steck T.L., Weinstein R.S. and Wallach D.F.H., "Inside-Out Red Cell Membrane Vesicles: Preparation and Purification", Science, 168, 1970, 255-257 hergestellt. Die Ery-RSO-Vesikel wurden in einem 5 mM PBS Puffer pH 8,0 (5 mM N₂HPO₄, 5 mM NH₂PO₄) dispergiert (im folgenden als Puffer B bezeichnet) und anschließend mittels Druckfiltration (Extrusion) durch Polykarbonatmembranen (100 nm Porendurchmesser) in kleine, einschalige Vesikel mit einem Durchmesser von 20-90 nm überführt. Zu 2 ml dieser Lösung (= 0.5 mg Gesamtprotein) wurde in einem Inkubationsgefäß der nach 1. modifizierte Objektträger zugegeben und für 5 h bei leichtem Schütteln inkubiert. Danach wurde der Träger dreimal mit Puffer B gewaschen. Der Erfolg der Beschichtung wurde mittels Infrarotspektroskopie in Reflexion dokumentiert. Eine schematische Darstellung der Anordnung der Membran auf dem Träger wird in Abbildung 1 gezeigt.

### 2.2 Membran des sarkoplasmatischen Retikulums

Aus dem Muskelgewebe eines Kaninchens wurden nach einem Verfahren von W. Hasselbach und M. Makinose (Biochem. Z. 1961, 333, 518-528) Membranvesikel des sarkoplasmatischen Retikulums hergestellt (SR-Vesikel) und in einem Puffer aus 100 mM Triethanolamin (pH=7,4) und 100 mM NaCl (Puffer C) dispergiert. Diese Dispersion wurde anschließend durch Ultraschallbehandlung in kleine, einschalige Vesikel mit einem Durchmesser von 20-90 nm überführt. Zu 2 ml dieser Lösung (≅ 0,5 mg Gesamtprotein) wurde in einem Inkubationsgefäß der nach 1. modifizierte Objektträger zugegeben und für 5 h bei leichtem Schütteln inkubiert. Danach wurde der Träger dreimal mit Puffer C gewaschen. Der Erfolg der Beschichtung wurde mittels Infrarotspektroskopie in Reflexion dokumentiert.

### 3. Elektrophorese (eindimensional)

### 3.1 Erythrozytenmembran

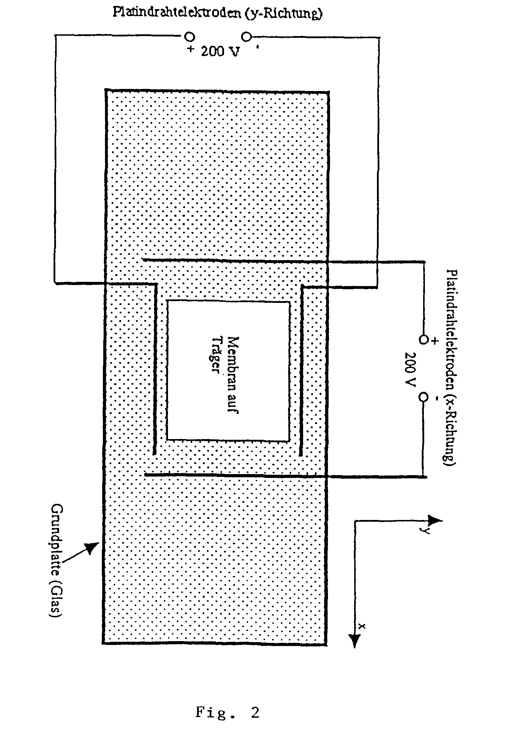

Die nach 2.1 präparierte Erythrozytenmembran wurde in die Meßkammer eines Fluoreszenzmikroskops eingebracht. Auf der Grundplatte (Glas) der Meßkammer waren 2 Elektrodenpaare aus Platindraht so angebracht, daß elektrische Felder sowohl in x-Richtung als auch in y-Richtung geschaltet werden konnten (Abbildung 2). Die präparierte Membran wurde auf der Grundplatte zwischen den 2 Elektrodenpaaren fixiert. Ein Deckel aus Teflon mit einem Glasfenster versiegelte über einen O-Ring diesen Bereich einschließlich der Elektroden wasserdicht nach außen, so daß die Membran vollständig von Puffer A umgeben war. Die Thermostatierung der Meßkammer ermöglichte während des gesamten Experimentes eine konstante Temperatur von 25°C. Einlaß- und Auslaßstutzen im aufsetzbaren Deckel der Meßkammer ermöglichten das Einspülen von Puffer und darin gelösten Molekülen während der Messung. Glasfenster in der Meßkammer ermöglichten die mikroskopische Beobachtung der Membran in Transmission.

Ein elektrisches Feld von 200 V/cm wurde angelegt und nach einer Dauer tₑ = 5 min wieder abgeschaltet. Unmittelbar nach Abschalten wurden monoklonale Primärantikörper gegen das zytoplasmatische Bindungsepitop von Bande-3 Protein (Mouse IgG, Sigma) und Glykophorin (Isotyp IgG1, Kappa, Dako) in die Meßkammer eingespült und für 5 min inkubiert. Anschließend wurde die Meßkammer mit der zehnfachen Menge ihres Eigenvolumens mit Puffer A gespült, um nicht gebundene Antikörper zu entfernen. Anschließend wurden für die beiden primären Antikörper spezifische fluoreszenzmarkierte sekundäre Antikörper (Monoclonal Antibody (Rat) to Mouse Isotyp IgG1 Kappa, mit FITC-Label und Goat Anti Mouse IgG mit TRITC-Label) in die Zelle gespült und die Inkubations- und Spülprozedur nach obigem Protokoll wiederholt. Die Zeitdauer zwischen dem Einspülen der proteinspezifischen Antikörper und dem Auswaschen der verbleibenden fluoreszenzmarkierten sekundären Antikörper betrug maximal 30 Minuten. Anschließend wurde der Objektträger mit Membran und den daran gebundenen Antikörpern bei 50°C und leichtem Vakuum getrocknet, um eine weitere (thermisch getriebene) Diffusion der Proteine samt Antikörper nach Abschalten der elektrischen Felder zu verhindern. Danach wurde die Verteilung der Antikörper auf der Oberfläche des Objektträgers fluoreszenzmikroskopisch untersucht. Der Einsatz von Kantenfiltern ermöglichte hierbei die Unterscheidung der Antikörperfluoreszenz der an Glykophorin bzw. Bande-3 gebundenen sekundären Antikörper. Durch digitale Bildverarbeitung wurde ein Densitogramm der jeweiligen Fluoreszenzintensitätsverteilung entlang einer beliebig gewählten, 10 µm breiten Verbindungslinie zwischen den beiden Elektroden aufgezeichnet.

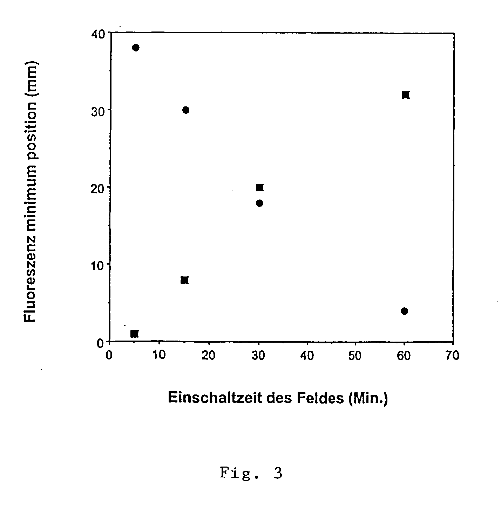

Die Versuche wurden für unterschiedliche Dauer der Einschaltung des elektrischen Feldes (tₑ = 5, 15, 30, 60 min.) bei unveränderter Feldstärke für jeweils neue, entsprechend 2.1 präparierte Membranproben wiederholt. Die Analyse der Meßergebnisse (Densitogramme als Funktion von tₑ) erfolgte durch die Bestimmung der Positionen xₘᵢₙ des Fluoreszenzminimums und xₘₐₓ des Fluoreszenzmaximums entlang der die beiden Elektroden verbindenden Linie in x-Richtung (sh. Abbildung 1). Diese Koordinaten wurden für xₘᵢₙ. in einem Diagramm als Funktion von tₑ aufgetragen und sind in Abbildung 3 gezeigt. Hierbei entspricht die Position xₘᵢₙ = 0 mm dem einen Rand des Objektträgers und Xₘᵢₙ = 40 mm dem gegenüberliegenden Rand. Es ist deutlich zu erkennen, daß sich beide Proteine durch das elektrische Feld in Richtung auf die Elektroden zu bewegt haben und daß die durch dieses Feld verursachte Driftgeschwindigkeit für beide Proteine unterschiedlich war.

Kontrollmessungen wurden für ein analog präpariertes System nach tₑ = 60 min. durchgeführt, bei der die elektrische Feldstärke jedoch nur 5 V/cm betrug.

In diesem Fall wurde keine signifikante Verschiebung der beiden Proteine mit der oben beschriebenen Technik nachgewiesen.

### 3.2 Membran des sarkoplasmatischen Retikulums (SR)

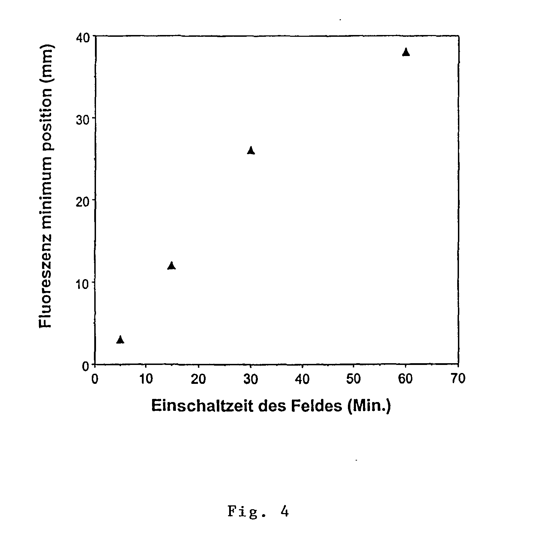

Meßaufbau und Durchführung war analog zu 3.1, gemessen wurde jedoch mit der unter 2.2 präparierten SR-Membran und unter Verwendung von Puffer C anstelle von Puffer B. Außerdem wurden in diesem Fall spezifische, primäre Antikörper für das zytoplasmatische Bindungsepitop der Kalzium-ATP-ase des SR (monoklonaler Mouse IgG 3-911, 912 (Dianova)) zum Einspülen nach Abschalten des elektrischen Feldes verwendet. Die Detektion der Antikörperverteilung erfolgte durch fluoreszenzmarkierte sekundäre Antikörper (Monoclonal Antibody (Rat) to Mouse Isotyp IgG1 Kappa, mit FITC-Label). Die Auswertung von Xₘᵢₙ als Funktion von tₑ zeigte eine deutliche Wanderung der Kalzium-ATP-ase in Richtung auf eine Elektrode (Abbildung 4). Hierbei entspricht die Position xₘᵢₙ = 0 mm dem einen Rand des Objektträgers und xₘᵢₙ = 40 mm dem gegenüberliegenden Rand.

Kontrollmessungen analog zu denen in 3.1 beschriebenen brachten auch hier keinen Hinweis auf eine Verschiebung des Proteins bei einer Feldstärke von nur 5 V/cm.

### 4. Elektrophorese (zweidimensional)

Diese Versuche wurden mit einem, zum in Abschnitt 3. beschriebenen, analogen Versuchsaufbau durchgeführt. Als einziger Unterschied wurden in diesen Experimenten zusätzlich die in Abbildung 1 gezeigten Elektroden in Querrichtung (Querelektroden, y-Richtung) angesteuert, die einen Aufbau eines zweiten elektrischen Feldes mit Feldrichtung senkrecht zum ersten Feld (Längselektroden, x-Richtung) gestatteten. Diese Versuche wurden mit den unter 3.2 beschriebenen SR-Membranen durchgeführt. Zunächst wurde der Versuch völlig analog zu Abschnitt 3.2 mittels der Längselektroden bei tₑ = 60 min. so durchgeführt, als wäre das zweite Paar Elektroden (Querelektroden) überhaupt nicht vorhanden. Nach dieser Zeit hatte sich die in der Membran vorhandene Kalzium-ATP-ase weitgehend in der Umgebung einer der beiden Längselektroden angereichert, während es an der anderen nicht mehr nachweisbar war (sh. Abschnitt 3.2). Nun wurde die Polung der Längselektroden umgekehrt und gleichzeitig die Querelektroden eingeschaltet (Spannung beider Elektrodenpaare 200 V/cm). Nach einer Zeit tₑ = 60 min. wurden beide Elektroden gleichzeitig ausgeschaltet. Anschließend wurde das in 3.1 bzw. 3.2 beschriebene Verfahren zur Sichtbarmachung der Kalzium-ATP-ase durch Einspülen des spezifischen Antikörpers sowie des fluoreszenzmarkierten sekundären Antikörpers und anschließender Auswaschung nicht gebundener Antikörper analog durchgeführt, der Träger samt Membran und Antikörper getrocknet und fluoreszenzmikroskopisch untersucht.

Die Untersuchung erbrachte eine weitgehende Lokalisation des Proteins in einer Ecke des durch die vier Elektroden aufgespannten Quadrates. Die Größe des Proteinflecks wurde mit 50-100 µm bestimmt. Dieses Ergebnis ist ein signifikanter Unterschied zur in 3.2 beschriebenen eindimensionalen Elektrophorese, wo das Protein nach derselben Zeit relativ homogen entlang einer der zwei Längselektroden verteilt war. Damit ist nachgewiesen, daß durch zweidimensionale Elektrophorese ein Membranprotein innerhalb der planaren Membran auf dem Objekträger so manipuliert werden kann, daß es schließlich an einem, durch zwei Dimensionen begrenzten Ort angereichert wird.

## Patentansprüche

1. Verfahren zur Auftrennung von Membranproteinen (membrandurchspannende oder partiell in die Membran insertierende Proteine), **dadurch gekennzeichnet, daß** in einem ersten Schritt die Oberfläche einer festen Phase (Trägermaterial) durch chemische Modifikation in einen Zustand versetzt wird, welcher in einem zweiten Schritt die Adsorption einer biologischen Membran auf der besagten modifizierten Oberfläche in einer Weise gestattet, dass die in der biologischen Membran enthaltenen Membranproteine ihre Fähigkeit zur Diffusion in der Membranebene auch nach der Adsorption weitgehend beibehalten und in einem dritten Schritt durch das Anlegen geeigneter elektrischer Felder an die nun trägerfixierte biologische Membran eine elektrophoretische Bewegung (Elektrophorese) der Membranproteine in der Membranebene ermöglicht wird, die schließlich zu ihrer Separation in einer oder zwei Dimensionen führt.

2. Verfahren nach Anspruch 1, **dadurch gekennzeichnet, dass** der besagte Träger eine planare Oberfläche aufweist und die besagte chemische Modifikation mit anschließender Adsorption einer biologischen Membran und Elektrophorese auf dieser planaren Oberfläche durchgeführt wird.

3. Verfahren nach Anspruch 1 oder 2, **dadurch gekennzeichnet, dass** der besagte feste Träger aus Silikatverbindungen, insbesondere Glas, aus Keramiken, aus Polymeren, aus Metallen oder aus Halbleitermaterialien besteht.

4. Verfahren nach einem der vorhergehenden Ansprüche, **dadurch gekennzeichnet, dass** die besagte chemische Modifizierung durch die Verfahrensschritte
a) Funktionalisieren der Oberfläche mit Molekülen zur Ausbildung einer im wesentlichen entweder hydrophoben oder hydrophilen Schicht und
b) Adsorption oder Chemisorption von mit dieser Schicht wechselwirkenden Molekülen
erfolgt.

5. Verfahren nach einem der vorhergehenden Ansprüche, **dadurch gekennzeichnet, dass** die besagte chemische Modifizierung die folgenden Verfahrensschritte umfaßt:
a) Funktionalisieren der Festkörperoberfläche und/oder
b) Adsorption oder Chemisorption von wechselwirkenden Molekülen und
c) Adsorption oder Chemisorption von weiteren Molekülen, die mit denen in Schritt b) verwendeten Molekülen zu Wechselwirkung fähig sind.

6. Verfahren nach Anspruch 4 oder 5, **dadurch gekennzeichnet, dass** die besagte Funktionalisierung der Trägeroberfläche durch das Aufbringen von Aminofunktionen, Epoxyfunktionen, Halogenalkylfunktionen und/oder Thiofunktionen vorgenommen wird, wobei insbesondere die Trägeroberfläche mit aminofunktionellen, epoxyfunktionellen, halogenalkylfunktionellen und/oder thiofunktionellen Molekülen behandelt wird.

7. Verfahren nach einem der Ansprüche 4 bis 6, **dadurch gekennzeichnet, dass** für die Funktionalisierung Silane, Mercaptane, und/oder Disulfide, insbesondere Alkyldisulfide eingesetzt werden.

8. Verfahren nach einem der Ansprüche 4 bis 7, **dadurch gekennzeichnet, dass** als wechselwirkende Moleküle Polymere, insbesondere Polyelektrolyte, Polyampholyte, vorzugsweise Proteine, und/oder Polyzwitterionen eingesetzt werden.

9. Verfahren nach einem der Ansprüche 4 bis 8, **dadurch gekennzeichnet, dass** als wechselwirkende Moleküle Polystyrolsulfonat und/oder Poly(styrol-co-Maleinsäureanhydrid) eingesetzt werden.

10. Verfahren nach einem der vorhergehenden Ansprüche, **dadurch gekennzeichnet, dass** die Trägeroberfläche durch technische Verfahren gezielt mikro- oder nanostrukturiert wurde.

11. Verfahren nach einem der vorhergehenden Ansprüche, **dadurch gekennzeichnet, dass** die Aufbringung der besagten biologischen Membran durch Fusion von Vesikeln dieser Membran auf der besagten modifizierten Oberfläche erfolgt.

12. Verfahren nach einem der vorhergehenden Ansprüche, **dadurch gekennzeichnet, dass** die Aufbringung der besagten biologischen Membran auf die modifizierte Oberfläche durch den Austausch von Vesikeln dieser Membran mit bereits auf der Oberfläche befindlichen Membranen oder Membranfragmenten erfolgt.

13. Verfahren nach einem der vorhergehenden Ansprüche, **dadurch gekennzeichnet, dass** die biologische Membran im wesentlichen aus Organellen eukaryotischer Zellen besteht.

14. Verfahren nach einem der vorhergehenden Ansprüche, **dadurch gekennzeichnet, dass** die Herstellung der Vesikel aus besagter biologischer Membran durch Ultraschallbehandlung oder durch das Pressen der biologischen Membran unter erhöhtem Druck durch nanoporöse Filter (Extrusion) und/oder durch Düsen erfolgt.

15. Verfahren nach einem der vorhergehenden Ansprüche, **dadurch gekennzeichnet, dass** die besagte Elektrophorese durch mindestens ein Paar Elektroden durchgeführt wird, welche ein elektrisches Feld erzeugen können, das im wesentlichen in der Ebene der besagten Membran liegt.

16. Verfahren nach Anspruch 15, **dadurch gekennzeichnet, dass** für die Elektrophorese mehr als ein Paar Elektroden verwendet werden, die elektrische Felder in jeder Raumrichtung erzeugen können.

17. Verfahren nach Anspruch 15 oder 16, **dadurch gekennzeichnet, dass** die Elektroden mit Gleichspannung oder mit Wechselspannung betrieben werden.

18. Verfahren nach einem der Ansprüche 15 bis 17, **dadurch gekennzeichnet, dass** die Elektrodenpaare gleichzeitig oder abwechselnd mit Spannung versorgt werden.

19. Verfahren nach einem der Ansprüche 15 bis 18, **dadurch gekennzeichnet, dass** die Elektroden zur Freisetzung von elektrischen Feldpulsen verwendet werden.

20. Verfahren nach einem der vorhergehenden Ansprüche, **dadurch gekennzeichnet, dass** die erreichte Separation der Membranproteine durch Fluoreszenz, insbesondere Einzelphotonendetektionstechniken, durch Lumineszenz oder durch radioaktive Markierung sichtbar gemacht bzw. detektiert wird.

21. Verfahren nach einem der vorhergehenden Ansprüche, **dadurch gekennzeichnet, dass** die erreichte Separation der Membranproteine durch massenspektrometrische Verfahren, insbesondere MALDI-TOF, nachgewiesen bzw. analysiert wird.

22. Verfahren nach einem der vorhergehenden Ansprüche, **dadurch gekennzeichnet, dass** zur Sichtbarmachung der Auftrennung von zuvor radioaktiv markierten Membranproteinen die Mehrfach-Photonen-Detektionstechnik (MPD) eingesetzt wird.

## Claims

1. A method of separating membrane proteins (proteins that extend through a membrane or partially insert into a membrane), **characterized in that**, in a first step, the surface of a solid phase (support material) is put by chemical modification into a state which, in a second step, permits the adsorption of a biological membrane on the said modified surface such that the membrane proteins in the biological membrane substantially retain their ability to diffuse in the membrane plane after adsorption and, in a third step, suitable electric fields are applied to the then support- fixed biological membrane to cause the membrane proteins to execute an electrophoretic movement (electrophoresis) in the membrane plane that finally leads to their separation in one or two dimensions.

2. A method according to claim 1, **characterized in that** the said support has a planar surface and the said chemical modification with subsequent adsorption of a biological membrane and electrophoresis is carried out on this planar surface.

3. A method according to claim 1 or 2, **characterized in that** the said solid support consists of silicate compounds, in particular glass, of ceramics, of polymers, of metals or of semiconductor materials.

4. A method according to any one of the preceding claims, **characterized in that** the said chemical modification is effected by the steps of
a) functionalizing the surface with molecules to form an essentially either hydrophobic or hydrophilic layer and
b) adsorbing or chemisorbing molecules interacting with this layer.

5. A method according to any one of the preceding claims, **characterized in that** the said chemical modification comprises the steps of:
a) functionalizing the solid state surface and/or
b) adsorbing or chemisorbing interacting molecules and
c) adsorbing or chemisorbing further molecules capable of interacting with the molecules used in step b).

6. A method according to claim 4 or 5, **characterized in that** the said functionalizing of the support surface is effected by applying amino functions, epoxy functions, haloalkyl functions and/or thio functions, in particular the support surface being treated with amino-functional, epoxy-functional, haloalkyl-functional and/or thio-functional molecules.

7. A method according to any one of claims 4 to 6, **characterized in that** the functionalizing utilizes silanes, mercaptans and/or disulphides, in particular alkyl disulphides.

8. A method according to any one of claims 4 to 7, **characterized in that** interacting molecules comprise polymers, in particular polyelectrolytes, polyampholytes, preferably proteins, and/or polyzwitterions.

9. A method according to any one of claims 4 to 8, **characterized in that** interacting molecules comprise polystyrene sulphonate and/or poly(styrene-co-maleicanhydride).

10. A method according to any one of the preceding claims, **characterized in that** the support surface was specifically micro- or nanostructured by technical processes.

11. A method according to any one of the preceding claims, **characterized in that** the applying of the said biological membrane is effected by fusion of vesicles of this membrane on the said modified surface.

12. A method according to any one of the preceding claims, **characterized in that** the applying of the said biological membrane to the modified surface is effected through the exchange of vesicles of this membrane with membranes or membrane fragments already present on the surface.

13. A method according to any one of the preceding claims, **characterized in that** the biological membrane consists essentially of organelles of eukaryotic cells.

14. A method according to any one the preceding claims, **characterized in that** the vesicles are formed from the said biological membrane by ultrasonic treatment or by pressing the biological membrane under elevated pressure through nanoporous filters (extrusion) and/or through nozzles.

15. A method according to any one of the preceding claims, **characterized in that** the said electrophoresis is carried out using at least one pair of electrodes capable of producing an electric field essentially located in the plane of the said membrane.

16. A method according to claim 15, **characterized in that** the electrophoresis utilizes more than one pair of electrodes capable of generating electric fields in every direction in space.

17. A method according to claim 15 or 16,
**characterized in that** the electrodes are operated with direct current voltage or with alternating current voltage.

18. A method according to any one of claims 15 to 17, **characterized in that** the electrode pairs are simultaneously or alternately supplied with voltage.

19. A method according to any one of claims 15 to 18, **characterized in that** the electrodes are used for releasing electric field pulses.

20. A method according to any one of the preceding claims, **characterized in that** the separation achieved for the membrane proteins is visualized or detected by fluorescence, particularly single photon detection techniques, by luminescence or by radioactive labelling.

21. A method according to any one of the preceding claims, **characterized in that** the separation achieved for the membrane proteins is detected or analysed by mass-spectrometric methods, in particular MALDI-TOF.

22. A method according to any one of the preceding claims, **characterized in that** the separation of previously radioactively labelled membrane proteins is visualized using multiple photon detection technology (MPD).

## Revendications

1. Procédé de séparation de protéines membranaires (protéines recouvrant la membrane ou partiellement insérées dans la membrane), **caractérisé en ce que** dans une première étape, la surface d'une phase solide (matériau de support) est transformée par modification chimique en un état qui autorise dans une deuxième étape l'adsorption d'une membrane biologique sur ladite surface modifiée de telle sorte que les protéines membranaires contenues dans la membrane biologique conservent largement leur capacité de diffusion dans la surface membranaire également après l'adsorption et dans une troisième étape, par l'application d'un champ électrique approprié sur la membrane biologique à présent fixée au support, un mouvement électrophorétique (électrophorèse) des protéines membranaires est permis dans la surface membranaire, qui entraîne finalement leur séparation dans une ou dans deux dimensions.

2. Procédé selon la revendication 1, **caractérisé en ce que** ledit support présente une surface planaire et ladite modification chimique est réalisée avec une adsorption consécutive d'une membrane biologique et l'électrophorèse est réalisée sur cette surface planaire.

3. Procédé selon la revendication 1 ou 2, **caractérisé en ce que** ledit support solide est constitué de composés de silicate, en particulier de verre, de céramiques, de polymères, de métaux ou de matériaux semi-conducteurs.

4. Procédé selon l'une quelconque des revendications précédentes, **caractérisé en ce que** ladite modification chimique s'effectue par les étapes du procédé comprenant :
a) la fonctionnalisation de la surface avec des molécules pour former une couche essentiellement hydrophobe ou hydrophile et
b) l'adsorption ou la chémisorption de ces molécules interagissant avec cette couche.

5. Procédé selon l'une quelconque des revendications précédentes, **caractérisé en ce que** ladite modification chimique comprend les étapes de procédé suivantes :
a) la fonctionnalisation de la surface du solide et/ou
b) l'adsorption ou la chémisorption de molécules interagissantes et
c) l'adsorption ou la chémisorption d'autres molécules qui sont capables d'interagir avec les molécules utilisées à l'étape b).

6. Procédé selon la revendication 4 ou 5, **caractérisé en ce que** ladite fonctionnalisation de la surface de support est entreprise par l'application de fonctions amino, de fonctions époxy, de fonctions halogéno-alkyle et/ou de fonctions thio, moyennant quoi en particulier la surface de support est traitée avec des molécules amino-fonctionnelles, époxy-fonctionnelles, halogéno-alkyl-fonctionnelles et/ou thio-fonctionnelles.

7. Procédé selon l'une quelconque des revendications 4 à 6, **caractérisé en ce que** pour la fonctionnalisation, on utilise des silanes, mercaptanes, et/ou disulfures, en particulier des alkyl-disulfures.

8. Procédé selon l'une quelconque des revendications 4 à 7, **caractérisé en ce que** l'on utilise comme molécules interagissantes, des polymères, en particulier, des poly-électrolytes, des polyampholytes, de préférence, des protéines et/ou des poly-zwitterions.

9. Procédé selon l'une quelconque des revendications 4 à 8, **caractérisé en ce que** l'on utilise comme molécules interagissantes, le polystyrène-sulfonate et/ou le poly(styrène-co-anhydride d'acide maléique).

10. Procédé selon l'une quelconque des revendications précédentes, **caractérisé en ce que** la surface de support a été micro-structurée ou nano-structurée de façon ciblée par des procédés techniques.

11. Procédé selon l'une quelconque des revendications précédentes, **caractérisé en ce que** l'application de ladite membrane biologique s'effectue par fusion des vésicules de cette membrane sur ladite surface modifiée.

12. Procédé selon l'une quelconque des revendications précédentes, **caractérisé en ce que** l'application de ladite membrane biologique s'effectue sur la surface modifiée par l'échange de vésicules de cette membrane avec des membranes ou des fragments de membrane se trouvant déjà sur la surface.

13. Procédé selon l'une quelconque des revendications précédentes, **caractérisé en ce que** la membrane biologique est constituée essentiellement d'organelles de cellules eucaryotes.

14. Procédé selon l'une quelconque des revendications précédentes, **caractérisé en ce que** la production des vésicules à partir de ladite membrane biologique s'effectue par traitement ultrasonore ou par compression de la membrane biologique sous une pression élevée au travers de filtres nanoporeux (extrusion) et/ou de buses.

15. Procédé selon l'une quelconque des revendications précédentes, **caractérisé en ce que** ladite électrophorèse est réalisée par au moins une paire d'électrodes qui peuvent générer un champ électrique qui se situe essentiellement à la surface de ladite membrane.

16. Procédé selon la revendication 15, **caractérisé en ce que** l'on utilise pour l'électrophorèse, plus d'une paire d'électrodes qui peuvent générer le champ électrique dans chaque direction spatiale.

17. Procédé selon la revendication 15 ou 16, **caractérisé en ce que** les électrodes sont actionnées avec une tension de courant continu ou une tension de courant alternatif.

18. Procédé selon l'une quelconque des revendications 15 à 17, **caractérisé en ce que** les paires d'électrodes sont alimentées par le courant de manière concomitante ou alternative.

19. Procédé selon l'une quelconque des revendications 15 à 18, **caractérisé en ce que** les électrodes sont utilisées pour la libération des pulsations de champ électrique.

20. Procédé selon l'une quelconque des revendications précédentes, **caractérisé en ce que** la séparation réalisée des protéines membranaires est rendue visible ou détectée par fluorescence, en particulier par des techniques de détection à photon unique, par luminescence ou par marquage radioactif.

21. Procédé selon l'une quelconque des revendications précédentes, **caractérisé en ce que** la séparation des protéines membranaires réalisée est détectée ou analysée par un procédé de spectrométrie de masse, en particulier de MALDI-TOF.

22. Procédé selon l'une quelconque des revendications précédentes, **caractérisé en ce que** pour visualiser la séparation des protéines membranaires radioactivement marquées au préalable, on utilise la technique de détection à photons multiples (MPD).
